(19) Europäisches Patentamt
European Patent Office
Office européen des brevets

(11) Publication number: **0 164 038**
**B1**

# (12) EUROPEAN PATENT SPECIFICATION

(45) Date of publication of patent specification: **18.10.89**

(51) Int. Cl.[4]: **C 07 C 13/50**, C 07 C 5/10, C 10 M 105/02 // C10N40:04

(21) Application number: **85106429.5**

(22) Date of filing: **24.05.85**

## (54) 2-decalyl-2-cyclohexyl derivatives of propane, process for their preparation, and their use.

(30) Priority: **05.06.84 JP 113916/84**

(43) Date of publication of application: **11.12.85 Bulletin 85/50**

(45) Publication of the grant of the patent: **18.10.89 Bulletin 89/42**

(84) Designated Contracting States: **DE FR GB IT**

(58) References cited:
**DE-A-1 543 978**
**GB-A-2 123 849**
**US-A-3 349 140**

(73) Proprietor: **IDEMITSU KOSAN COMPANY LIMITED**
**No. 1-1, 3-chome, Marunouchi Chiyoda-ku**
**Tokyo (JP)**

(72) Inventor: **Tsubouchi, Toshiyuki**
**No. 1660, Kamiizumi Sodegaura-machi**
**Kimitsu-gun Chiba-ken (JP)**
Inventor: **Shimizu, Nobuaki**
**No. 1205-139, Kamiizumi Sodegaura-machi**
**Kimitsu-gun Chiba-ken (JP)**

(74) Representative: **Türk, Dietmar, Dr. rer. nat. et al**
**Türk, Gille + Hrabal Patentanwälte Bruckner Strasse 20**
**D-4000 Düsseldorf 13 (DE)**

**The file contains technical information submitted after the application was filed and not included in this specification**

**EP 0 164 038 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

The present invention relates to new 2-decalyl-2-cyclohexyl-derivatives of propane having the general formula

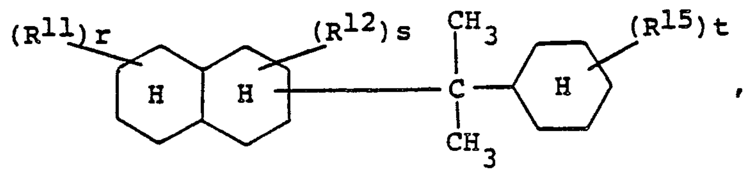

(I)

wherein each of the symbols $R^{11}$, $R^{12}$ and $R^{15}$ represents a hydrogen atom or an alkyl group having 1 to 4 carbon atoms and each of the suffixes r, s and t is an integer of 1, 2 or 3, a process for the preparation of these compounds as well as their use as a base stock for a fluid for traction drive having a remarkably increased traction coefficient.

A fluid for traction drive is employed in a variety of traction drive apparatuses such as continuously variable transmissions. Along with the requirement for a smaller and more compact design, apparatuses of this type for power transmission are required to be capable of being driven at a high velocity and under a high load as a trend in recent years. Therefore, it is eagerly desired to develop a high-performance fluid for traction drive capable of withstanding severer and severer working conditions.

Various compounds have been hitherto proposed as usable as a fluid for traction drive of which those compounds having two or more naphthenic rings in a molecule are known to belong to a class of preferable ones.

In GB—A—2 123 849 substituted alkanes of a similar structure and methods for the preparation thereof as well as their use as traction drive fluids is disclosed. Although the generic formulae in the said reference encompassed a vast number of compounds, the present 2-decalyl-2-cyclohexyl propanes of the above formula (I) are not disclosed therein. The only compound specifically disclosed in this reference and having a relation-ship to the presently claimed compounds are ethane derivatives having methyl substituents at the decalyl group. Also, there is no suggestion in the said reference that the 2-substituted propane compounds of the present invention are especially useful or even perform better as traction drive fluids than the ethane compounds specifically disclosed therein.

The inventors also have since long pertained extensively to the investigations for the development of a high-performance fluid for traction drive and proposed several compounds, of which it has been proved that a compound represented by the structural formula

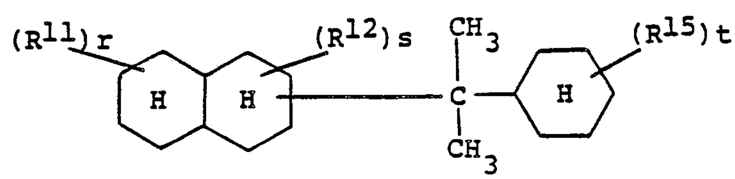

(I)

in which each of the symbols $R^{11}$, $R^{12}$ and $R^{15}$ denotes a hydrogen atom or an alkyl group having 1 to 4 carbon atoms and each of the suffixes r, s and t is an integer of 1, 2 or 3, is particularly preferable as a fluid for traction drive with a remarkably large traction coefficient, in particular, at elevated temperatures.

On the way of the development works of the above mentioned compound usable as a base stock of a fluid for traction drive, the inventors have become aware that the performance of the compound as a fluid for traction drive depends considerably on the conditions of the manufacturing process or, in particular, of the step of the hydrogenation reaction to give different values of the traction coefficient.

Summary of the Invention

It is therefore an object of the present invention to provide novel compounds represented by the above given general formula (I) and having a large traction coefficient with a remarkably small temperature dependence with reproducibility.

Another object of the invention is to provide a method in which the compound represented by the general formula (I) can be imparted with a remarkably and reproducibily increased traction coefficient by virtue of the improvement in the conditions of the hydrogenation reaction or, in particular, proper selection of the catalyst used in the hydrogenation reaction.

Therefore, the present invention relates to new 2-decalyl-2-cyclohexyl derivatives of propane useful as a fluid for traction drive and represented by the general formula

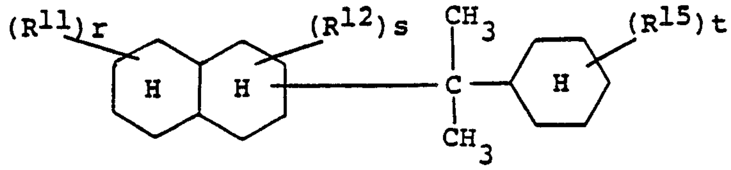 , (I)

in which each of the symbols $R^{11}$, $R^{12}$ and $R^{15}$ denotes a hydrogen atom or an alkyl group having from 1 to 4 carbon atoms and each of the suffices r, s and t is an integer of 1, 2 or 3.

A particularly preferred compound of the above general formula (I) is 2-decahydronaphthyl-3-cyclohexyl propane of the formula

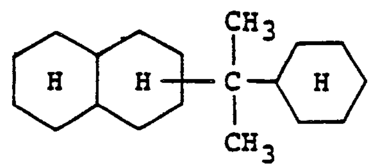

Furthermore, the present invention relates to a process for the preparation of 2-decalyl-2-cyclohexyl derivatives of propane having the general formula

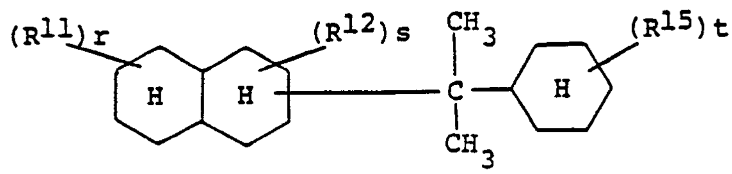 , (I)

in which each of the symbols $R^{11}$, $R^{12}$ and $R^{15}$ denotes a hydrogen atom or an alkyl group having from 1 to 4 carbon atoms and each of the suffixes r, s and t is an integer of 1, 2 or 3, which comprises catalytically hydrogenating a compound represented by the general formula

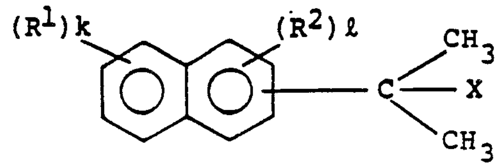 (II)

or

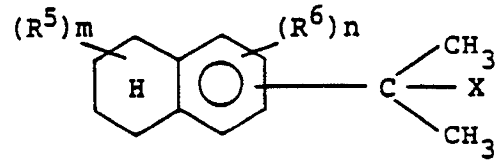 (III),

in which each of the symbols $R^1$, $R^2$, $R^5$ and $R^6$ denotes a hydrogen atom or an alkyl group having from 1 to 4 carbon atoms, X

is a group expressed by the formula 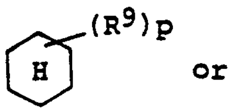 or 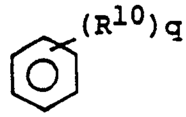 ,

$R^9$ and $R^{10}$ each being a hydrogen atom or an alkyl group having from 1 to 4 carbon atoms, and each of the suffixes k, l, m, n, p and q is an integer of 1, 2 or 3, in the presence of a catalyst containing a platinum group metal selected from the class consisting of ruthenium, platinum, rhodium and iridium.

According to a preferred embodiment of the present invention 2-decahydronaphthyl-2-cyclohexyl propane of the formula

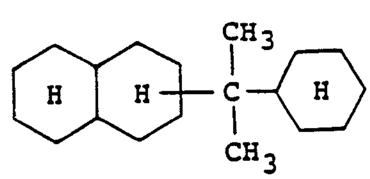

according to the above process wherein a compound represented by the above general formula (II) or (III), in which $R^1$, $R^2$, $R^5$, $R^6$, $R^9$ and $R^{10}$ each denote a hydrogen atom, is catalytically hydrogenated.

The hydrogenation reaction preferably is carried out at a temperature of from 10 to 300°C, in particular 50 to 200°C, and at a pressure of from 1 to 200 bar, in particular of from 5 to 50 bar.

Finally, the present invention relates to the use of a compound of the above formula (I) as a base stock for a fluid for traction drive having a better traction co-efficient, in particular at elevated temperatures, than the 2-substituted ethanes known from GB—A—2 123 849.

Brief Description of the Drawing

Fig. 1 and 2 graphically show the traction coefficient of the compounds prepared in the example and comparative examples as a function of the temperature.

Detailed Description of the Preferred Embodiments

The compound represented by the general formula (I) can be prepared by the catalytic hydrogenation of the compound represented by the general formula (II) or (III). The catalyst used in this hydrogenation reaction is a catalyst of a platinum group metal selected from the class consisting of ruthenium, platinum, rhodium and iridium and these active ingredients of the catalyst are, in most cases, supported on a conventional catalyst carrier such as silica, alumina, active carbon although the form of the catalyst is not limited thereto. Preferable reaction conditions in the hydrogenation treatment include a reaction temperature in the range from 10 to 300°C, preferably from 50 to 200°C and a reaction pressure in the range from 1 to 200 bar, preferably from 5 to 50 bar.

The compound represented by the formula (I) obtained by the method of the present invention has a remarkably increased traction coefficient in comparison with those obtained by the hydrogenation using other conventional catalysts such as nickel so that this compound is very excellent as a fluid for traction drive. The reason therefor is presumably that the decahydronaphthalene derivatives as the reaction product of the inventive method contain a predominant amount of the cis-isomeric molecules in respect of the configuration of the decahydronaphthalene nucleus in which the hydrogen atoms at the 9- and 10-positions are in the cis-orientation relative to each other.

The compound obtained by the method of the invention can be used as such as a base stock of a fluid for traction drive having a large traction coefficient with a remarkably small temperature dependence thereof in a wide range of temperature. Therefore, traction drive apparatuses using the fluid prepared by the inventive method can be designed remarkably compactly and are capable of running even under very severe operation conditions of high temperatures and high loads so that they are applicable to a wide variety of machines such as continuously variable transmissions for automobilies and industrial machines and hydraulic machines.

In the following, the present invention is described in more detail by way of an Example and Comparative Examples, in which the determination of the traction coefficients was performed using a two roller friction tester having two independently rotatable discs of the same size of 52 mm diameter and 6 mm thickness contacting with each other. These two discs were rotated at a constant velocity of 2000 r.p.m. and 1900 r.p.m. respectively under a contacting pressure of 70 kg load by means of a spring. By determining the torque using a strain gage and a torque meter from which the traction coefficient was calculated. Each of the discs was made of a bearing steel SUJ—2 and the surface thereof was finished by buffing using an alumina abrasive of 0.03 μm particle diameter to have a surface roughness of $R_{max}$ = 0.2 μm. The temperature of the lubricating oil during measurement was varied in the range from 60°C to 140°C by heating the oil holder with a heater.

Comparative Example 1

Into a glass flask of 3 liter capacity were introduced 1000 g (7.58 mole) of tetrahydronaphthalene and 300 g of concentrated sulfuric acid and the mixture was cooled at 0°C on ice-sodium chloride bath. Then, 400 g (3.85 mole) of styrene were added dropwise into the mixture under stirring over a period of 3 hours followed by further stirring for additional one hour to complete the reaction. The mixture was kept standing and the separated organic layer was washed with 500 ml of a 1N aqueous solution of sodium hydroxide and followed by 500 ml of a saturated aqueous solution of sodium chloride, three times and dried with anhydrous sodium sulfate. The organic mixture was distilated under reduced pressure to give 750 g of a fraction boiling at 135 to 148°C under a pressure of 0,2 mbar. The analysis of this fraction gave a result that the fraction was mainly composed of a mixture of 1-(5-(1,2,3,4-tetrahydronaphthyl)-1-phenyl ethane and 1-(6-(1,2,3,4-tetrahydronaphthyl))-1-phenyl ethane.

In the next place, a 500 g portion of the above obtained fraction was introduced into an autoclave of 1 liter capacity together with 50 g of an alumina-supported hydrogenation catalyst containing 0.5% by weight of platinum and the hydrogenation reaction was performed for 4 hours at a reaction temperature of 200°C under a hydrogen pressure of 50 bar. After cooling, the reaction mixture was filtrated to remove the catalyst and the filtrate was analyzed after stripping off lighter matters to find that the hydrogenation reaction had proceeded to more than 99.9% and that the product was mainly composed of a mixture of 1-(1-decahydronaphthyl)-1-cyclohexyl ethane and 1-(2-decahydronaphthyl)-1-cyclohexyl ethane. This product had properties including a specific gravity of 0.94 (15/4°C), kinematic viscosities of 34 centistokes at

40°C and 4.6 centistokes at 100°C and a refractive index $n_D^{20}$ of 1.5040. The content of the cis-isomer therein was 71%. The traction coefficient of this product was determined in the temperature range from 60°C to 140°C and the results are shown in FIGURE 1 of the accompanying drawing.

Comparative Example 2

The experimental procedure was substantially the same as in Comparative Example 1 except that the platinum catalyst used in the hydrogenation reaction was replaced with 20 g of 5% ruthenium-carbon, the reaction temperature was decreased to 120°C and the hydrogen pressure was decreased to 20 bar. The thus obtained product had properties including a specific gravity of 0.94 (15/4°C), kinematic viscosities of 39 centistokes at 40°C and 4.9 centistokes at 100°C and a refractive index $n_D^{20}$ of 1.5048. The content of the cis-isomers was 88%. The traction coefficient of this product is shown in FIGURE 1 as a function of the temperature in the range from 60°C to 140°C.

Comparative Example 3

The experimental procedure was substantially the same as in Comparative Example 1 except that the platinum catalyst used in the hydrogenation reaction was replaced with 15 g of 5% rhodium-carbon and the reaction temperature was decreased to 80°C. The thus obtained product had properties including a specific gravity of 0.94 (15/4°C), kinematic viscosities of 37 centistokes at 40°C and 4.7 centistokes at 100°C and a refractive index $n_D^{20}$ of 1.5047. The content of the cis-isomers was 86%. The traction coefficient of this product is shown as a function of the temperature in the range from 60°C to 140°C.

Comparative Example 4

A mixture composed of 1-(1-naphthyl)-1-phenyl ethane and 1-(2-naphthyl)-1-phenyl ethane was prepared in the same manner as in the reaction of tetrahydronaphthalene and styrene described in Comparative Example 1 except that 1000 g of tetrahydronaphthalene were replaced with 1000 g of naphthalene with further addition of 3000 ml of carbon tetrachloride.

The thus obtained reaction product mixture was subjected to the hydrogenation reaction in the same manner as in Comparative Example 1 except that the platinum catalyst was replaced with 10 g of the same ruthenium catalyst as used in Comparative Example 2 with addition of 10 g of water, the hydrogen pressure was increased to 70 bar and the reaction temperature was decreased to 150°C. The thus obtained product had properties including a specific gravity of 0.94 (15/4°C), kinematic viscosities of 38 centistokes at 40°C and 4.9 centistokes at 100°C and a refractive index $n_D^{20}$ of 1.5050. The content of the cis-isomers was 83%. The traction coefficient of this product is shown in FIGURE 1 as a function of the temperature in the range from 60°C to 140°C.

Example

Dry hydrogen chloride gas was blown into 590 g of α-methylstyrene contained in a glass flask of 1 liter capacity at room temperature under stirring to give 750 g of cumyl chloride. After, 2000 g of tetrahydronaphthalene and 70 g of titanium tetrachloride were introduced into a glass flask of 5 liter capacity, the mixture which was cooled at 0°C on ice-sodium chloride bath and then a mixture of 300 g of tetrahydronaphthalene and 550 g of the cumyl chloride was added dropwise into the mixture in the flask under agitation over a period of 3 hours followed by further stirring for additional 1 hour to complete the reaction. The reaction mixture after the post-treatment in the same manner as in Comparative Example 1 was distilled under reduced pressure to give 400 g of a fraction boiling at 133 to 140°C under a pressure of 0.04 mbar. This fraction was identified by analysis to be mainly 2-(6-(1,2,3,4-tetrahydronaphthyl))-2-phenyl propane.

Thereafter, 400 g of this product were introduced into an autoclave of 1 liter capacity together with 30 g of the same ruthenium catalyst as used in Comparative Example 2 and the hydrogenation reaction was performed at 150°C for 4 hours under a hydrogen pressure of 50 bar. After cooling and post-treatment undertaken in the same manner as in Comparative Example 1, the reaction product was analyzed to find that the hydrogenation reaction had proceeded to an extent of 99.9% or more and it was identified to be 2-decahydronaphthyl-2-cyclohexyl propane. This product had properties including a specific gravity of 0.94 (15/4°C), kinematic viscosities of 131 centistokes at 40°C and 7.7 centistokes at 100°C and a refractive index $n_D^{20}$ of 1.5105. The content of the cis-isomer was 90%. The traction coefficient of the product is shown in FIGURE 2 as a function of the temperature in the range from 60°C to 140°C.

Comparative Example 5

The experimental procedure was substantially the same as in Comparative Example 1 except that the platinum catalyst used in the hydrogenation reaction was replaced with 50 g of a nickel catalyst for hydrogenation. The thus obtained product had properties including a specific gravity of 0.94 (15/4°C), kinematic viscosities of 29 centistokes at 40°C and 4.2 centistokes at 100°C and a refractive index $n_D^{20}$ of 1.5025. The content of the cis-isomers was 42%. The traction coefficient of this product is shown in FIGURE 1 as a function of the temperature in the range from 60°C to 140°C.

Comparative Example 6

The experimental procedure was substantially the same as in Comparative Example 4 except that the ruthenium catalyst used in the hydrogenation reaction was replaced with 50 g of the same nickel catalyst for hydrogenation as used in Comparative Example 5, the reaction temperature was increased to 250°C and the hydrogen pressure was increased to 100 bar. The thus obtained product had properties including a specific gravity of 0.93 (15/4°C), kinematic viscosities of 27 centistokes at 40°C and 4.0 centistokes at 100°C and a refractive index $n_D^{20}$ of 1.5020. The content of the cis-isomer was 35%. The traction coefficient of the product is shown in FIGURE 1 as function of the temperature in the range from 60°C to 140°C.

Comparative Example 7

The experimental procedure was substantially the same as in the Example except that the ruthenium catalyst used in the hydrogenation was replaced with 50 g of the same nickel catalyst for hydrogenation as used in Comparative Example 5 and the reaction temperature was increased to 250°C. The thus obtained product had properties including a specific gravity of 0.94 (15/4°C), kinematic viscosities of 120 centistokes at 40°C and 7.4 centistokes at 100°C and a refractive index $n_D^{20}$ of 1.5097. The content of the cis-isomer was 40%. The traction coefficient of the product is shown in FIGURE 2 as a function of the temperature in the range from 60°C to 140°C.

**Claims**

1. A process for the preparation of a compound useful as a fluid for traction drive represented by the general formula

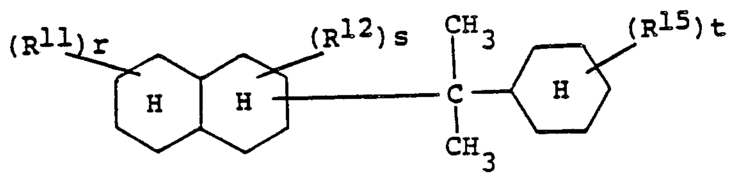 , (I)

in which each of the symbols $R^{11}$, $R^{12}$ and $R^{15}$ denotes a hydrogen atom or an alkyl group having from 1 to 4 carbon atoms and each of the suffixes r, s and t is an integer of 1, 2 or 3, which comprises catalytically hydrogenating a compound represented by the general formula

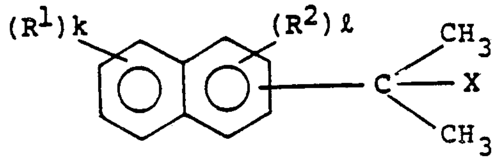 (II)

or

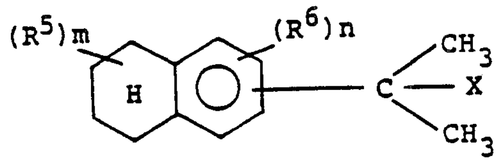 (III),

in which each of the symbols $R^1$, $R^2$, $R^5$ and $R^6$ denotes a hydrogen atom or an alkyl group having from 1 to 4 carbon atoms, X is a group expressed by the formula

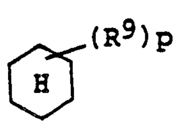 or 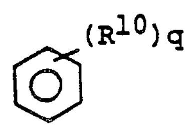 ,

$R^9$ and $R^{10}$ each being a hydrogen atom or an alkyl group having from 1 to 4 carbon atoms, and each of the suffixes k, l, m, n, p and q is an integer of 1, 2 or 3, in the presence of a catalyst containing a platinum group metal selected from the class consisting of ruthenium, platinum, rhodium and iridium.

2. The process according to Claim 1, wherein a compound represented by the general formula (II) or (III), in which $R^1$, $R^2$, $R^5$, $R^6$, $R^9$ and $R^{10}$ each denote a hydrogen atom, is catalytically hydrogenated.

3. The process according to Claim 1 or 2, wherein the hydrogenation reaction is carried out at a temperature of from 10 to 300°C and at a pressure of from 1 to 200 bar.

4. The process according to Claim 1 or 2, wherein the hydrogenation reaction is carried out at a temperature of from 50 to 200°C and at a pressure of from 5 to 50 bar.

5. A compound useful as a fluid for traction drive represented by the general formula

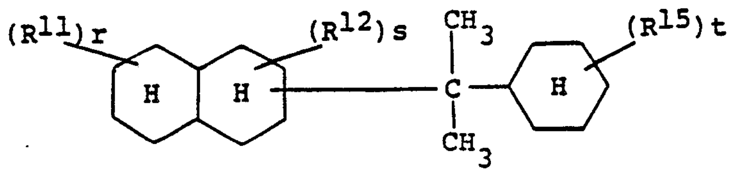 , (I)

in which each of the symbols $R^{11}$, $R^{12}$ and $R^{15}$ denotes a hydrogen atom or an alkyl group having from 1 to 4 carbon atoms and each of the suffixes r, s and t is an integer of 1, 2 or 3.

6. The compound of claim 5, which is 2-decahydronaphtyl-2-cyclohexyl propane of the formula

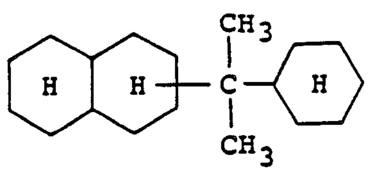

7. Use of a compound of formula (I) as claimed in claim 5 as a base stock for a fluid for traction drive.

**Patentansprüche**

1. Verfahren zur Herstellung einer Verbindung geeignet als eine Flüssigkeit für Kraftmaschine, dargestellt durch die allgemeine Formel

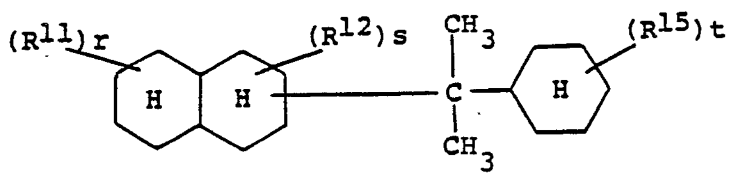 , (I)

worin die Symbole $R^{11}$, $R^{12}$ und $R^{15}$ jeweils ein Wasserstoffatom oder eine Alkylgruppe mit von 1 bis 4 Kohlenstoffatomen bedeuten und die Suffixe r, s und t jeweils eine ganze Zahl von 1, 2 oder 3 sind, welches umfaßt katalytische Hydrierung einer Verbindung, dargestellt durch die allgemeine Formel

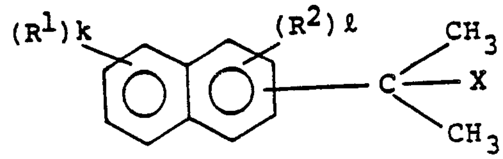 (II)

oder

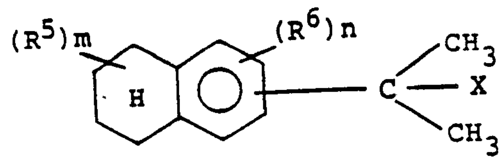 (III),

worin die Symbole $R^1$, $R^2$, $R^5$ und $R^6$ jeweils ein Wasserstoffatom oder eine Alkylgruppe mit von 1 bis 4 Kohlenstoffatomen bedeuten, X eine Gruppe ist ausgedrückt durch die Formel

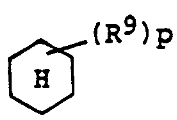 oder 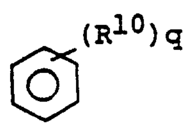 ,

wobei $R^9$ und $R^{10}$ jeweils ein Wasserstoffatom oder eine Alkylgruppe mit von 1 bis 4 Kohlenstoffatomen sind, und die Suffixe k, l, m, n, p und q jeweils eine ganze Zahl von 1, 2 oder 3 ist, in der Gegenwart eines

Katalysators enthaltend ein Metall der Platingruppe, ausgewählt aus der Gruppe bestehend aus Ruthenium, Platin, Rhodium und Iridium.

2. Verfahren nach Anspruch 1, worin eine Verbindung dargestellt durch die allgemeine Formel (II) oder (III) in der $R^1$, $R^2$, $R^5$, $R^6$, $R^9$ und $R^{10}$ jeweils ein Wasserstoffatom bedeuten, katalytisch hydriert wird.

3. Verfahren nach Anspruch 1 oder 2, worin die Hydrierungsreaktion bei einer Temperatur von 10 bis 300°C und bei einem Druck von 1 bis 200 Bar durchgeführt wird.

4. Verfahren nach Anspruch 1 oder 2, worin die Hydrierungsreaktion bei einer Temperatur von 50 bis 200°C und bei einem Druck von 5 bis 50 Bar durchgeführt wird.

5. Verbindung geeignet als eine Flüssigkeit für Kraftmaschine, dargestellt durch die allgemeine Formel

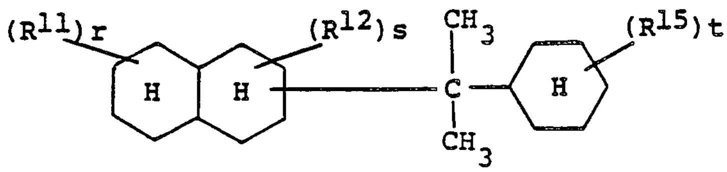 , (I)

worin die Symbole $R^{11}$, $R^{12}$ und $R^{15}$ jeweils ein Wasserstoffatom oder eine Alkylgruppe mit von 1 bis 4 Kohlenstoffatomen bedeuten und die Suffixe r, s und t jeweils eine ganze Zahl von 1, 2 oder 3 sind.

6. Verbindung nach Anspruch 5, die 2-Decahydronaphthyl-2-cyclohexylpropan der Formel

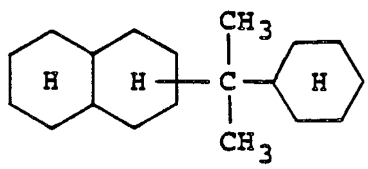

ist.

7. Verwendung einer Verbindung der Formel (I) nach Anspruch 5 als ein Basismaterial für eine Flüssigkeit für Kraftmaschine.

**Revendications**

1. Procédé pour la préparation d'un composé utilisable en tant que fluide de transmission, représenté par la formule générale

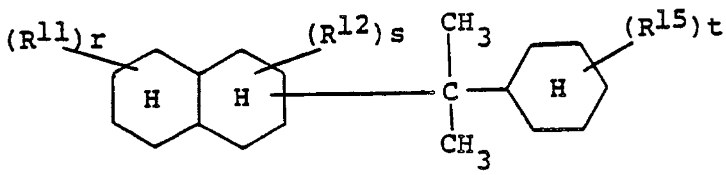 , (I)

dans laquelle chacun des symboles $R^{11}$, $R^{12}$ et $R^{15}$ représente un atome d'hydrogène ou un groupe alkyle ayant de 1 à 4 atomes de carbone, et chacun des indices r, s et t est un nombre entier valant 1, 2 ou 3, lequel comprend l'hydrogénation catalytique d'un composé représenté par la formule générale

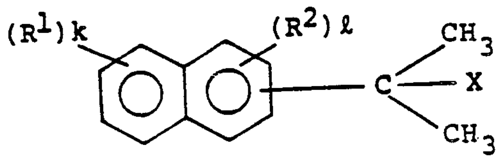 (II)

ou

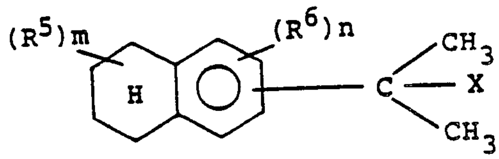 (III),

dans lesquelles formules chacun des symboles $R^1$, $R^2$, $R^5$ et $R^6$ représente un atome d'hydrogène ou un groupe alkyle ayant de 1 à 4 atomes de carbone, X est un groupe représenté par la formule

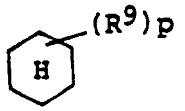

ou

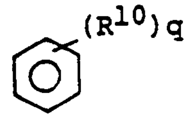

,

$R^9$ et $R^{10}$ étant chacun un atome d'hydrogène ou un groupe alkyle ayant de 1 à 4 atomes de carbone, et chacun des indices k, l, m, n, p et q étant un nombre entier valant 1, 2 ou 3, en présence d'un catalyseur contenant un métal de la famille du platine, choisi parmi le ruthénium, le platine, le rhodium et l'iridium.

2. Procédé selon la revendication 1, dans lequel un composé représenté par la formule générale (II) ou (III), dans lequel $R^1$, $R^2$, $R^5$, $R^6$, $R^9$ et $R^{10}$ représentent chacun un atome d'hydrogène, est soumis à l'hydrogènation catalytique.

3. Procédé selon la revendication 1 ou 2, dans lequel la réaction d'hydrogènation est effectuée à une température de 10 à 300°C et sous une pression de 1 à 200 bars.

4. Procédé selon la revendication 1 ou 2, dans lequel la réaction d'hydrogénation est effectuée à une température de 50 à 200°C et sous une pression de 5 à 50 bars.

5. Composé utilisable en tant que fluids de transmission, représenté par la formule générale

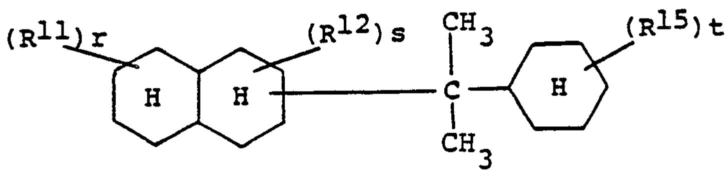

, (I)

dans laquelle chacun des symboles $R^{11}$, $R^{12}$ et $R^{15}$ représente un atome d'hydrogène ou un groupe alkyle ayant de 1 à 4 atomes de carbone, et chacun des indices r, s et t est un nombre entier valent 1, 2 ou 3.

6. Composé selon la revendication 5, lequel est le 2-décahydronaphtyl-2-cyclohexylpropane de formule

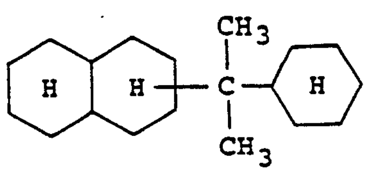

7. Utilisation d'un composé de formule (I) selon la revendication 5, en tant que matière première pour un fluide de transmission.

FIG. 1

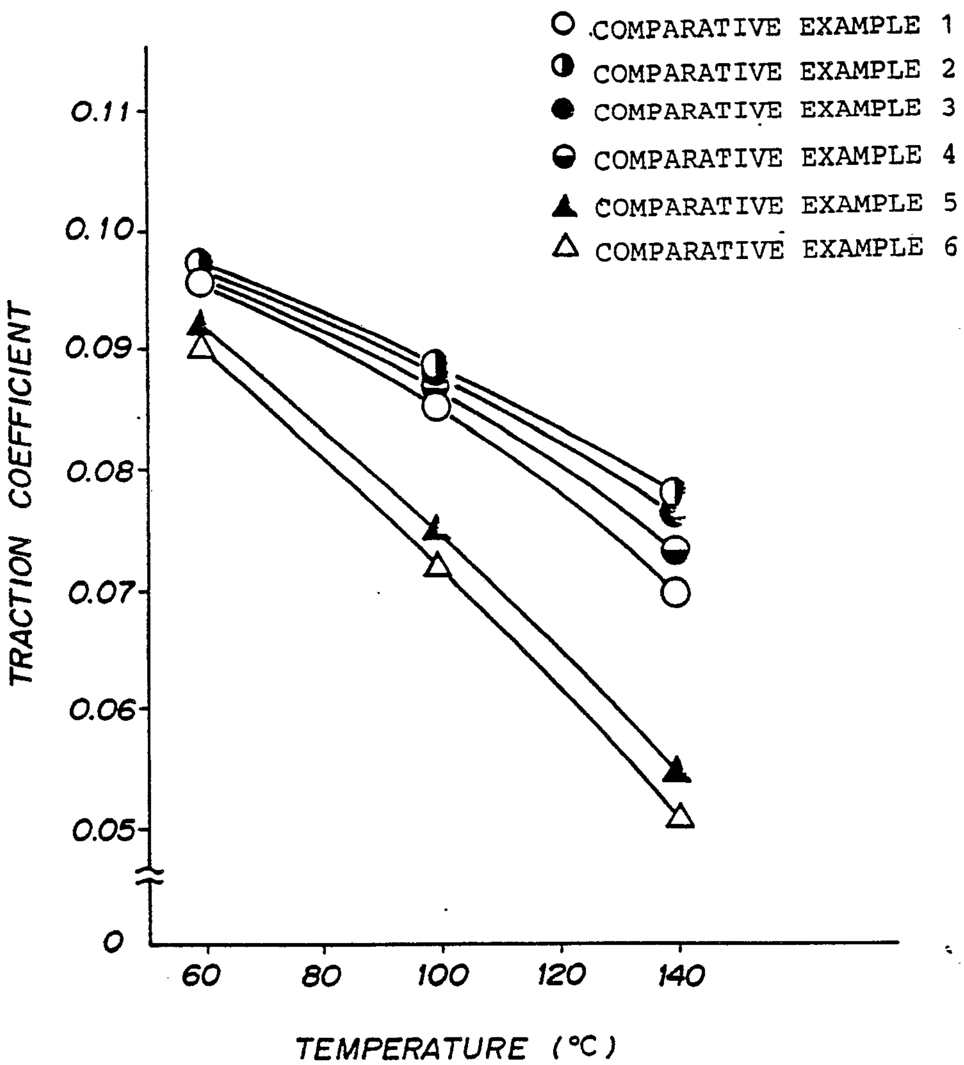
○ COMPARATIVE EXAMPLE 1
◐ COMPARATIVE EXAMPLE 2
● COMPARATIVE EXAMPLE 3
⊖ COMPARATIVE EXAMPLE 4
▲ COMPARATIVE EXAMPLE 5
△ COMPARATIVE EXAMPLE 6
0.11
0.10
0.09
0.08
0.07
0.06
0.05
0
TRACTION COEFFICIENT
60
80
100
120
140
TEMPERATURE (°C)

FIG. 2

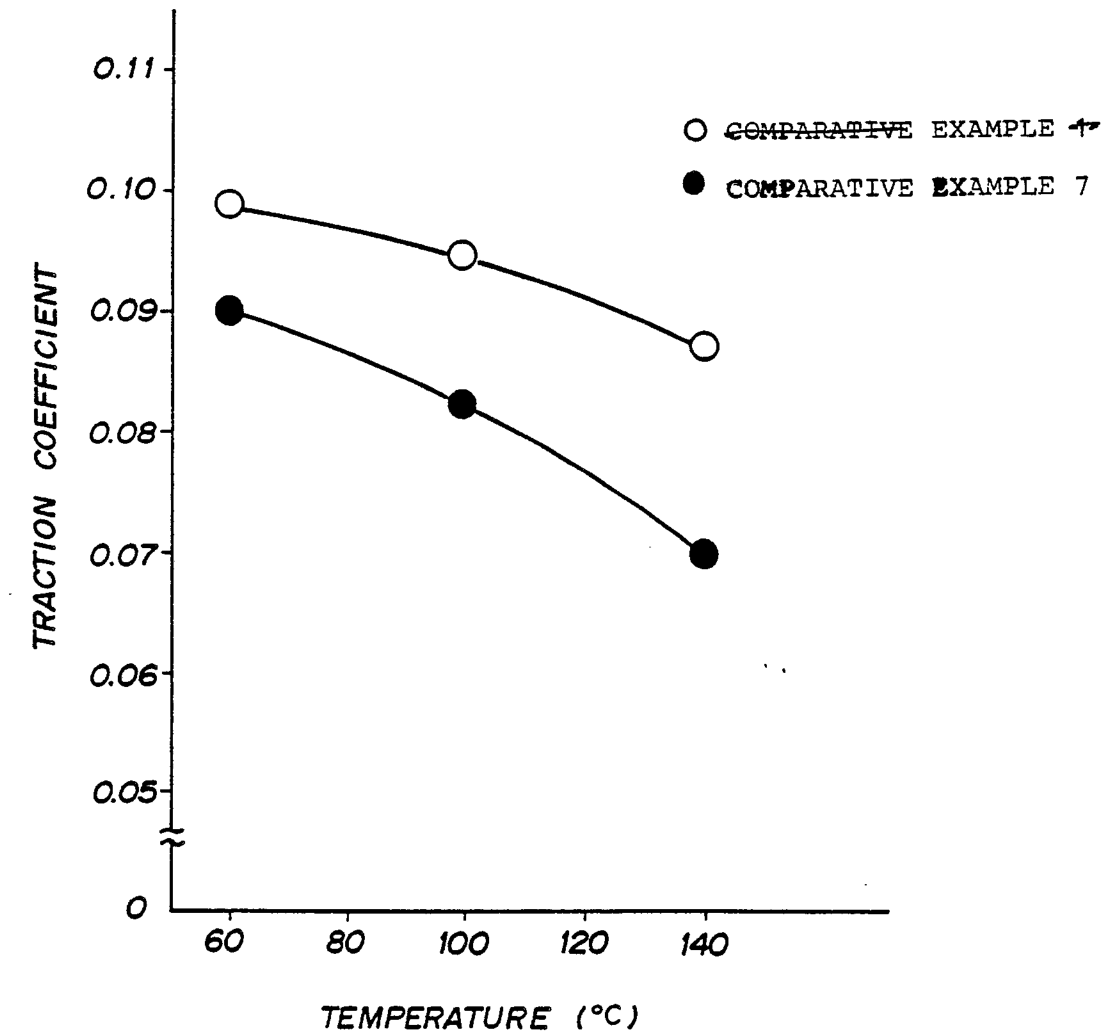
COMPARATIVE EXAMPLE 1
COMPARATIVE EXAMPLE 7
TRACTION COEFFICIENT
0.11
0.10
0.09
0.08
0.07
0.06
0.05
0
60
80
100
120
140
TEMPERATURE (°C)